# EUROPEAN PATENT APPLICATION

(11) **EP 1 908 743 A1**
(43) Date of publication of application: **09.04.2008**
(21) Application number: 06781605.8
(22) Date of filing: 25.07.2006
(51) Int. Cl.: C07C 2/02, B01J 23/06, B01J 23/26, B01J 23/46, B01J 27/02, B01J 29/18, B01J 29/40, B01J 29/70, B01J 29/85, C07C 2/86, C07C 4/08, C07C 13/28, C07C 15/14, C07B 61/00

(54) **(ALKYLPHENYL)ALKYLCYCLOHEXANE AND METHOD FOR PRODUCING (ALKYLPHENYL)ALKYLCYCLOHEXANE OR ALKYLBIPHENYL**

(30) Priority: 26.07.2005 JP 2005215382
(71) Applicant: MITSUBISHI GAS CHEMICAL COMPANY, INC., Chiyoda-ku, Tokyo 100-8324 (JP)
(72) Inventor: KANBARA, Y., c/o Mitsubishi Gas Chemical Co., Inc., Kita-ku,Niigata-shi, Niigata 950-3112 (JP); WATANABE, T., c/o Mitsubishi Gas Chemical Co., Inc, Chiyoda-ku, Tokyo (JP); MOROHASHI, K., c/o Mitsubishi Gas Chemical Co. Inc, Kita-ku, Niigata-shi, Niigata 950-3112 (JP); NISHIUCHI, J., c/o Mitsubishi Gas Chemical Co. Inc, Kurashiki-shi, Okayama 712-8525 (JP)
(74) Representative: Gille Hrabal Struck Neidlein Prop Roos
(86) International application number: PCT/JP2006/314693
(87) International publication number: WO 2007/013469

(57) **Abstract**

The invention provides a method for producing an (alkylphenyl)alkylcyclohexane, including a step of condensing an alkylbenzene with an alkylcyclohexene or an alkylcyclohexanol in the presence of an acid catalyst, and (alkylphenyl)alkylcyclohexane represented by formula (8). The (alkylphenyl)alkylcyclohexane produced through the production method can be transformed into an alkylbiphenyl, a biphenylpolycarboxylic acid, or a biphenylpolycarboxylic anhydride. Through the production method, an (alkylphenyl)alkylcyclohexane and an alkylbiphenyl of interest can be readily and selectively produced. 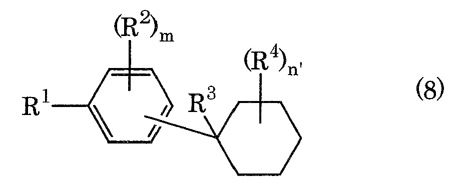 (wherein R¹ represents a C1-C4 alkyl group; R² represents a C1-C4 alkyl group; m is an integer of 0 to 2; n' is an integer of 2 to 5; other conditions are the same as defined in claim 18.)

## Description

### Technical Field

The present invention relates to a method for producing an (alkylphenyl)alkylcyclohexane including a step of condensing an alkylbenzene with an alkylcyclohexene or alkylcyclohexanol, and to a method for producing an alkylbiphenyl or its related products including a step of further dehydrogenating the (alkylphenyl)alkylcyclohexane. (Alkylphenyl)alkylcyclohexanes and alkylbiphenyls are important compounds in organic synthesis chemistry, and serve as starting materials for liquid crystals, pharmaceuticals, and monomers.
Among these compounds, 4,4'-biphenyldicarboxylic acid, which is produced through dehydrogenating and dealkylating 1-(4-methylphenyl)-1,4-dimethylcyclohexane represented by formula (1): 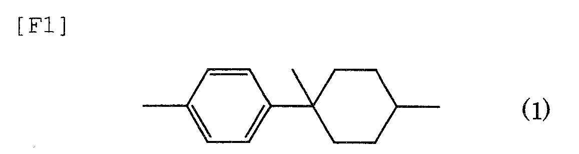 and oxidizing the formed 4,4'-dimethylbiphenyl, is a useful source for polyesters.
Also, 3,4,3',4'-biphenyltetracarboxylic anhydride, which is produced through dehydrogenating and dealkylating 1-(3,4-dimethylphenyl)-1,3,4-trimethylcyclohexane represented by formula (2): 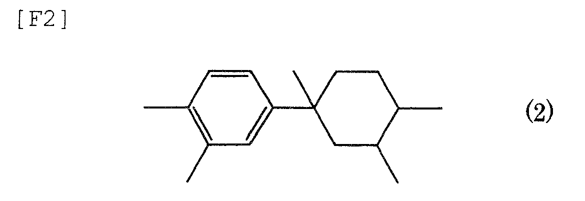 and oxidizing and dehydrating the formed 3,4,3',4'-tetramethylbiphenyl, is a useful source of heat-resistant polyimides.

### Background Art

Hitherto, there has never been reported a method for producing an (alkylphenyl)alkylcyclohexane represented by, for example formula (1) or (2), based on condensation of an alkylbenzene with an alkylcyclohexene or alkylcyclohexanol. One known method for producing 1-(4-methylphenyl)-1,4-dimethylcyclohexane employs 1,4-dimethyl-1-chlorocyclohexane instead of alkylcyclohexene, and involves subjecting the chlorocyclohexane to Friedel-Crafts condensation with toluene in the presence of aluminum chloride (Non-Patent Document 1). However, 1,4-dimethyl-1-chlorocyclohexane is not readily available, and expensive aluminum chloride must be used in an amount larger than the equivalent amount of the halogen compound. In addition, treatments of by-produced hydrogen chloride and waste aluminum chloride after reaction are cumbersome processes. Thus, this approach is not considered an industrially advantageous method.

Meanwhile, there have been known a variety methods for producing alkylbiphenyls. One method is condensation between bromoalkylbenzene and a Grignard reagent to form an alkylbiphenyl selectively. However, this method is not practically employed in the industry, due to low availability of starting materials. In a production method for alkylbiphenyl based on alkylation of biphenyl, a mixture of alkylbiphenyls is produced, and a target alkylbiphenyl is difficult to produce at high efficiency (Patent Document 1). In this way, there has not been reported an industrial method for producing an alkylbiphenyl of interest at high selectivity.

[Non-Patent Document 1] Azerb. khim. Zh. '67, 1, 69-71
[Patent Document 1] Japanese Patent Application Laid-Open (kokai) No. 4-5246

### Disclosure of the Invention

Under such circumstances, the present inventors have carried out extensive studies for producing (alkylphenyl)alkylcyclohexane and alkylbiphenyl in an industrially advantageous manner, and have found that an alkylbenzene is readily condensed with an alkylcyclohexene or an alkylcyclohexanol in the presence of an acid catalyst, to thereby yield an (alkylphenyl)alkylcyclohexane. The inventors have also found that through dehydrogenation and dealkylation of the thus-yielded (alkylphenyl)alkylcyclohexane, a target alkylbiphenyl can be readily produced at high selectivity. The present invention has been accomplished on the basis of these findings.

Accordingly, the present invention is directed to a method for producing an (alkylphenyl)alkylcyclohexane represented by formula (6): 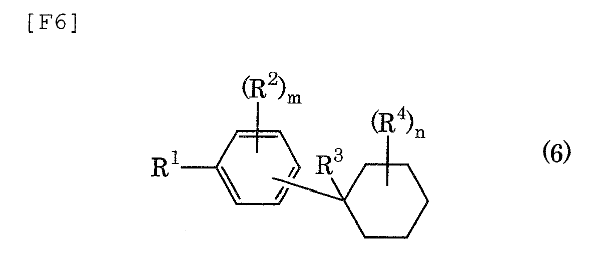 (wherein R¹ represents a C1-C4 alkyl group; R² represents a C1-C4 alkyl group which is identical to or different from R¹; R³ represents a C1-C4 alkyl group; R⁴ represents a C1-C4 alkyl group which is identical to or different from R³; m is an integer of 0 to 2; and n is an integer of 1 to 5, wherein when no R² is present or R² is present at the o-position with respect to R¹, the cyclohexane ring is present at the p-position with respect to R¹, and when R² is present at the m-position with respect to R¹, the cyclohexane ring is present at the m-position with respect to R¹), the method comprising a step of condensing an alkylbenzene represented by formula (3): 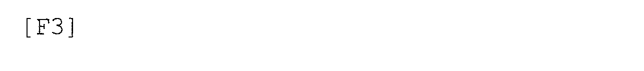 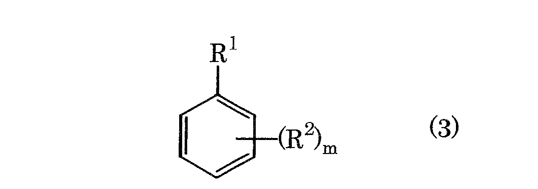 (wherein R¹ and m have the same meanings as defined above, wherein when m is 1, R² is present at the o- or m-position with respect to R¹, and when m is 2, two R²s are present at positions which are different from each other and are ortho with respect to R¹) with an alkylcyclohexene represented by formula (4): 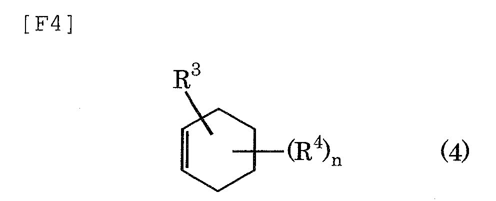 (wherein R³ and n have the same meanings as defined above, wherein when n is an integer of 2 to 5, a plurality of R⁴s may be identical to or different from one another, except that R³ and R⁴ or two R⁴s are bonded to one carbon atom) or with an alkylcyclohexanol represented by formula (5): 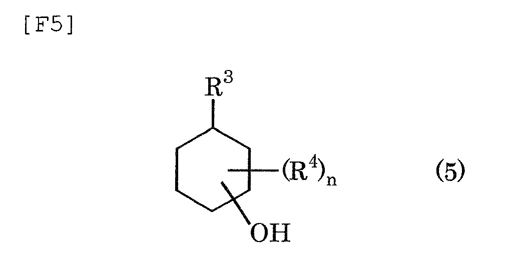 (wherein R³, R⁴, and n have the same meanings as defined above) in the presence of an acid catalyst.

The present invention is also directed to a method for producing an alkylbiphenyl represented by formula (7): 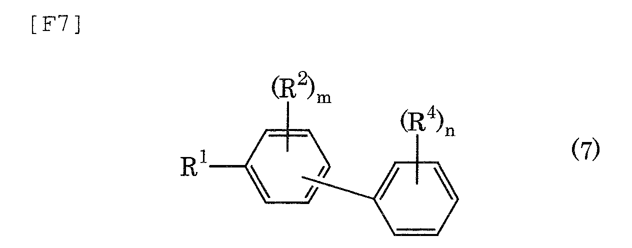 (wherein R¹, R², R⁴, m, and n have the same meanings as defined above, wherein when no R² is present or R² is present at the o-position with respect to R¹, the benzene ring having R⁴ is present at the p-position with respect to R¹, and when R² is present at the m-position with respect to R¹, the benzene ring having R⁴ is present at the m-position with respect to R¹), the method comprising dehydrogenating and dealkylatying the (alkylphenyl)alkylcyclohexane produced above in the presence of a catalyst.

The present invention is also directed to an (alkylphenyl)alkylcyclohexane represented by formula (8): 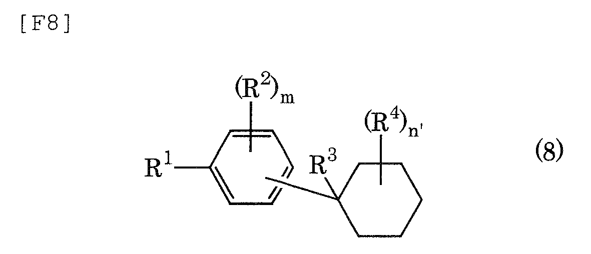 (wherein R¹, R², R³, R⁴, and m have the same meanings as defined above, and n' is an integer of 2 to 5, wherein when no R² is present or R² is present at the o-position with respect to R¹, the cyclohexane ring is present at the p-position with respect to R¹, and when R² is present at the m-position with respect to R¹, the cyclohexane ring is present at the m-position with respect to R¹).

### Brief Description of the Drawings

Fig. 1 is a ¹H-NMR spectrum of isomer 1 of 1-(3,4-dimethylphenyl)-1,3,4-trimethylcyclohexane produced in Example 7.
Fig. 2 is a ¹H-NMR spectrum of isomer 2 of 1-(3,4-dimethylphenyl)-1,3,4-trimethylcyclohexane produced in Example 7.

### Best Modes for Carrying Out the Invention

The method for producing an (alkylphenyl)alkylcyclohexane according to the present invention includes a step of condensing an alkylbenzene with an alkylcyclohexene or alkylcyclohexanol, in the presence of an acid catalyst.
The alkylbenzene is represented by formula (3): 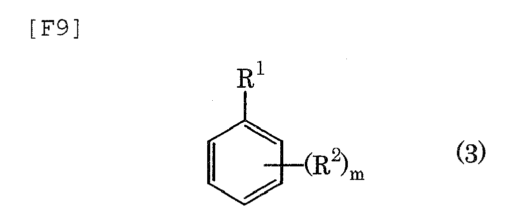 (wherein R¹ represents a C1-C4 alkyl group; R² represents a C1-C4 alkyl group which is identical to or different from R¹; and m is an integer of 0 to 2, wherein when m is 1, R² is present at the o- or m-position with respect to R¹, and when m is 2, two R²s are present at positions which are different from each other and are ortho with respect to R¹). Specific examples of the alkylbenzene include toluene, o-xylene, m-xylene, 1,2,3-trimethylbenzene, ethylbenzene, n-propylbenzene, isopropylbenzene, n-butylbenzene, t-butylbenzene, 1,2-methylethylbenzene, 1,3-methylethylbenzene, and 1,2-diethylbenzene. Of these, polymethylbenzenes having methyl groups as alkyl groups are preferred from the viewpoints of high reaction rate and material availability. Specifically, toluene, o-xylene, m-xylene, and 1,2,3-trimethylbenzene are particularly preferred.

The alkylcyclohexene is represented by formula (4): 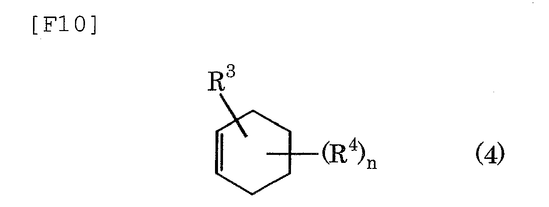 (R³ represents a C1-C4 alkyl group; R⁴ represents a C1-C4 alkyl group which is identical to or different from R³; and n is an integer of 1 to 5, wherein when n is an integer of 2 to 5, a plurality of R⁴s may be identical to or different from one another, except that R³ and R⁴ or two R⁴s are bonded to one carbon atom). Specific examples of the alkylcyclohexene include 1,2-dimethylcyclohexene, 1,3-dimethylcyclohexene, 1,4-dimethylcyclohexene, 1,2,3-trimethylcyclohexene, 1,2,4-trimethylcyclohexene, 1,3,5-trimethylcyclohexene, 1,2,3,4-tetramethylcyclohexene, 1,2,4,5-tetramethylcyclohexene, 1,2,3,4,5-pentamethylcyclohexene, 1,2,3,4,5,6-tetramethylcyclohexene, diethylcyclohexene, di-n-cyclohexene, diisopropylcyclohexene, di-n-butylcyclohexene, di-t-butylcyclohexene, 1,2-methylethylcyclohexene, 1,3-methylethylcyclohexene, and 1,4-methylethylcyclohexene. Of these, 1,2-dimethylcyclohexene, 1,3-dimethylcyclohexene, 1,4-dimethylcyclohexene, 1,2,4-trimethylcyclohexene, and 1,3,5-trimethylcyclohexene are preferred.

The alkylcyclohexene may be produced through a known method, for example, Diels-Alder reaction between a diene compound and an allyl compound, dehydration of an alkylcyclohexanol, or removal of hydrogen halide from a halocyclohexane. Alternatively, the alkylcyclohexene may be produced through catalytic partial hydrogenation of an alkylbenzene represented by formula (9): 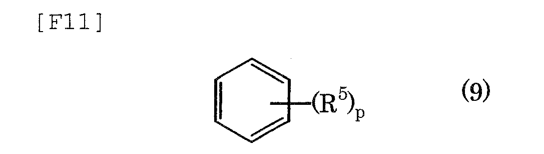 (wherein R⁵ represents a C1-C4 alkyl group, and p is an integer of 2 to 6, wherein a plurality of R⁵s may be identical to or different from one another).

Examples of the alkylbenzene represented by formula (9) include o-, m-, and p-xylenes, 1,2,3-trimethylbenzene, 1,2,4-trimethylbenzene (pseudocumene), 1,3,5-trimethylbenzene, 1,2,3,4-tetramethylbenzene, 1,2,4,5-tetramethylbenzene, 1,2,3,4,5-pentamethylbenzene, 1,2,3,4,5,6-tetramethylbenzene, diethylbenzene, di-n-propylbenzene, di-isopropylbenzene, din-butylbenzene, di-t-butylbenzene, 1,2-methylethylbenzene, 1,3-methylethylbenzene, and 1,4-methylethylbenzene. Of these, alkyulbenzenes in which R⁵ is a methyl group are preferred from the viewpoints of high reaction rate and material availability. Specifically, xylene (isomers) and trimethylbenzene (isomers) are more preferred, with m-xylene, p-xylene, and pseudocumene being particularly preferred.

Any catalyst which is imposed in generally performed hydrogenation can be employed in catalytic partial hydrogenation of the alkylbenzene represented by formula (9). Specific examples include metals such as ruthenium, rhodium, rhenium, platinum, palladium, nickel, cobalt, chromium, iridium, and copper. In use, these metals may be supported on a carrier such as carbon, alumina, silica, zirconia, hafnia, titania, or magnesia, or may be in the form of metallic microparticles. The amount (by relative weight) of catalyst is preferably 0.000001 to 10 with respect to alkylbenzene represented by formula (9), more preferably 0.00001 to 1. In order to enhance selectivity to alkylcyclohexene, a co-catalyst such as zinc sulfate, cobalt sulfate, barium sulfate, zinc chloride, zinc oxide, zinc bromide, zinc iodide, or zinc hydroxide may be used. The amount of co-catalyst (by relative weight) is preferably 0.00001 to 100 with respect to the catalyst employed, more preferably 0.0001 to 10. If deteriorated, the catalyst may be reactivated through a conventional method, for example, air passage at high temperature.

Catalytic partial hydrogenation of the alkylbenzene represented by formula (9) is preferably performed in a fixed bed in a tube reactor or a suspension bed in a bath reactor. In the case of a suspension bed in a bath reactor, water may be added to form an oil-water dual phase system, and alcohol or the like may also be added to the system. In this case, the ratio of water/alkylbenzene (formula (9)) by weight is preferably 0.001 to 100, more preferably 0.1 to 10. When the amount of water is excessively small, effect of water fails to be attained, whereas when it is excessive, space time yield decreases. The reaction temperature is preferably 0°C to 300°C, more preferably 50 to 200°C. When the temperature is excessively high, selectivity decreases, whereas when the temperature is excessively low, conversion does not increase. The hydrogen pressure is 0.1 to 30 MPa, preferably 1 to 15 MPa. When the pressure is excessively high, high-pressure apparatuses must be used, whereas when the pressure is excessively low, conversion does not increase. The reaction time is 10 minutes to 20 hours, when performed in a batch manner.

The alkylcyclohexene represented by formula (4) produced through the aforementioned hydrogenation process includes isomers in terms of stereo-relationship between the alkyl group and the double bond. Specifically, p-xylene produces two isomers: 1,4-dimethyl-1-cyclohexene and 1,4-dimethyl-2-cyclohexene, and 1,2,4-trimethylbenzene produces five isomers: 1,2,4-trimethyl-1-cyclohexene, 1,3,6-trimethyl--1-cyclohexene, 2,5,6-trimethyl-1-cyclohexene, 1,4,5-trimethyl-1-cyclohexene, and 2,3,5-trimethyl-1-cyclohexene. These isomers may be subjected to condensation with the alkylbenzene represented by formula (3) after isomer separation or without further separation. Alternatively, these isomers may be isomerized in the presence of an acid catalyst.

In hydrogenation of the alkylbenzene represented by formula (9), an alkylcyclohexane, in which the aromatic ring has been thoroughly hydrogenated, is by-produced. Alkylcyclohexene, alkylcyclohexane, and unreacted alkylbenzene may be separated through a conventional method such as distillation, extraction-distillation, fractionation, extraction, filtration, or crystallization. Unreacted alkylbenzene may be reused as a starting material for producing alkylcyclohexene.

In the method for producing an (alkylphenyl)alkylcyclohexane according to the present invention, instead of an alkylcyclohexene, an alkylcyclohexanol represented by formula (5): 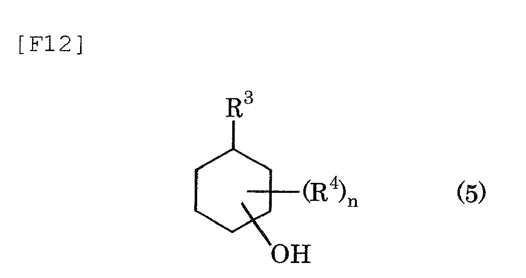 (wherein R³, R⁴, and n have the same meanings as defined above) may be condensed with an alkylbenzene represented by formula (3) in the presence of an acid catalyst. Specific examples of the alkylcyclohexanol include 1,2-dimethylcyclohexanol, 2,3-dimethylcyclohexanol, 3,4-dimethylcyclohexanol, 4,5-dimethylcyclohexanol, 5,6-dimethylcyclohexanol, 1,3-dimethylcyclohexanol, 1,4-dimethylcyclohexanol, 1,5-dimethylcyclohexanol, 1,6-dimethylcyclohexanol, 2,3-dimethylcyclohexanol, 2,4-dimethylcyclohexanol, 2,5-dimethylcyclohexanol, 2,6-dimethylcyclohexanol, 3,4-dimethylcyclohexanol, 3,5-dimethylcyclohexanol, 3,6-dimethylcyclohexanol, 4,5-dimethylcyclohexanol, 4,6-dimethylcyclohexanol, 1,2,3-trimethylcyclohexanol, 2,3,4-trimethylcyclohexanol, 3,4,5-trimethylcyclohexanol, 4,5,6-trimethylcyclohexanol, 1,3,4-trimethylcyclohexanol, 2,4,5-trimethylcyclohexanol, 3,5,6-trimethylcyclohexanol, 1,3,6-trimethylcyclohexanol, 2,5,6-trimethylcyclohexanol, 1,4,5-trimethylcyclohexanol, 3,4,6-trimethylcyclohexanol, 2,3,5-trimethylcyclohexanol, 1,3,5-trimethylcyclohexanol, 2,4,6-trimethylcyclohexanol, 1,2,3,4-tetramethylcyclohexanol, 1,2,3,5-tetramethylcyclohexanol, 1,2,3,6-tetramethylcyclohexanol, 1,2,4,5-tetramethylcyclohexanol, 1,2,3,4,5-pentamethylcyclohexanol, 1,2,3,4,5,6-hexamethylcyclohexanol, and these compounds in which the methyl groups have been substituted by ethyl, n-propyl, isopropyl, n-butyl, sec-butyl, or t-butylgroups. Of these, polymethylcyclohexanols are preferred from the viewpoints of high reaction rate and material availability. Specifically, dimethylhexanol and trimethylhexanol are more preferred. No particular limitation is imposed on the method for producing the alkylcyclohexanol. The alkylcyclohexanol may be produced through, for example, nucleus hydrogenation of an alkylphenol or hydration of an alkylcyclohexene.

The mole ratio of alkylbenzene to alkylcyclohexene is preferably 0.001 to 1,000, more preferably 0.1 to 100. When the mole ratio is excessively small, self-condensation of alkylcyclohexene tends to proceed, whereas when the mole ratio is in excess, it is not economically preferred. The mole ratio of alkylbenzene to alkylcyclohexanol is preferably 0.001 to 1,000, more preferably 0.1 to 100. For example, in condensation reaction between toluene and 1,4-dimethylcyclohexene, the toluene/1,4-dimethylcyclohexene mole ratio is preferably 2 to 20, more preferably 3 to 10. When the mole ratio falls within the range, self-condensation of 1,4-dimethylcyclohexene is prevented, to thereby increase selectivity to 1-(4-methylphenyl)-1,4-dimethylcyclohexane. In order to further enhance selectivity, preferably, 1,4-dimethylcyclohexene is gradually added to toluene. In condensation reaction between o-xylene and 1,2,4-trimethylcyclohexene, the o-xylene/1,2,4-trimethylcyclohexene mole ratio is preferably 1 to 100, more preferably 2 to 50. When the mole ration falls within the range, self-condensation of 1,2,4-trimethylcyclohexene is prevented, to thereby increase selectivity to 1-(3,4-dimethylphenyl)-1,3,4-trimethylcyclohexane. In order to further enhance selectivity, preferably, 1,2,4-trimethylcyclohexene is gradually added to o-xylene.

The condensation reaction may be performed in a solvent. The solvent employed in condensation is a compound which is inert to the condensation reaction. Examples of employable solvents include solvents generally employed in Friedel-Crafts reaction such as nitromethane, nitrobenzene, carbon disulfide, and acetonitrile; and aliphatic hydrocarbons such as hexane, cyclohexane, petroleum ether, octane, and decalin. Furthermore, alkylcyclohexane by-produced in the aforementioned production of alkylcyclohexene may also be used. An alkylbenzene in which the p-position (not the o-position) with respect to the alkyl group has been per-alkylated may also be used as a solvent. In contrast, an alkylbenzene in which the p-position with respect to the alkyl group has not been per-alkylated involves the condensation reaction, and such an alkylbenzene is not preferred as a solvent.

Examples of the acid catalyst employed in condensation reaction include protonic acids and Lewis acids such as sulfuric acid, hydrochloric acid, phosphoric acid, polyphosphoric acid, hydrogen fluoride, hydroborofluoric acid, boron trifluoride, aluminum trichloride, aluminum tribromide, gallium trichloride, gallium tribromide, iron trichloride, antimony pentachloride, tin tetrachloride, titanium tetrachloride, and zinc chloride. Alternatively, a cation-exchange resin such as Nafion or a solid acid such as silica-alumina or zeolite may also be used as a condensation catalyst. Examples of zeolite include A-type, ferrierite-type, ZSM-5 (MFI-type), ZSM-12 (MTW-type), mordenite-type, β-type, X-type, and Y-type. These species in which the silicate moiety has been substituted by a phosphate moiety, which have been cation (H⁺, NH₄⁺, metallic ion, etc.)-changed, or of which silica-alumina ratio has been modified may also be used. These acid catalysts may be used singly or in combination.

Depending on the species of the alkylcyclohexene employed as a starting material, the use of the aforementioned acid catalyst may result in by-production of isomers of non-interest in considerable amounts. Specifically, when condensation between toluene and 1,4-dimethylcyclohexene is performed, in addition to 1-(4-methylphenyl)-1,4-dimethylcyclohexane, isomers such as 2-(4-methylphenyl)-1,4-dimethylcyclohexane (formula (10)), 1-(3-methylphenyl)-1,4-dimethylcyclohexane (formula (11)), and 2-(3-methylphenyl)-1,4-dimethylcyclohexane (formula (12)) may be by-produced. In this case, use of sulfuric acid as a catalyst results in production of the target 1-(4-methylphenyl)-1,4-dimethylcyclohexane at high yield. 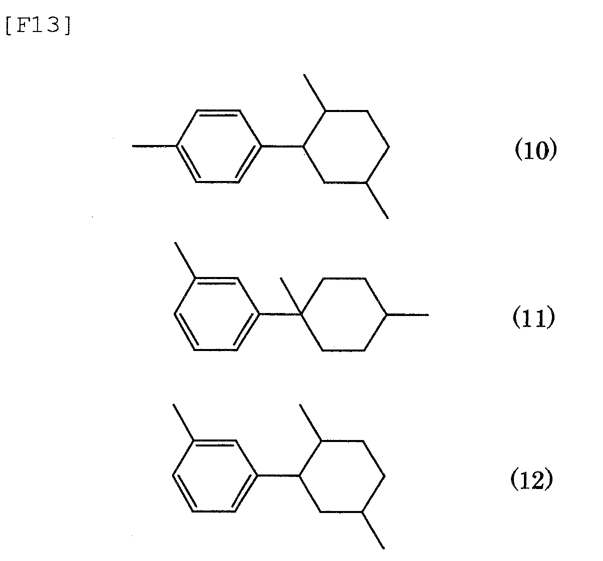 When condensation between o-xylene and 1,2,4-trimethylcyclohexene is performed, in addition to 1-(3,4-dimethylphenyl)-1,3,4-trimethylcyclohexane, isomers such as 1-(3,4-dimethylphenyl)-1,2,5-trimethylcyclohexane (formula (13)) and 1-(3,4-dimethylphenyl)-1,2,4-trimethylcyclohexane (formula (14)) may be by-produced. 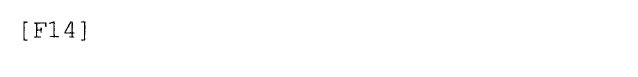 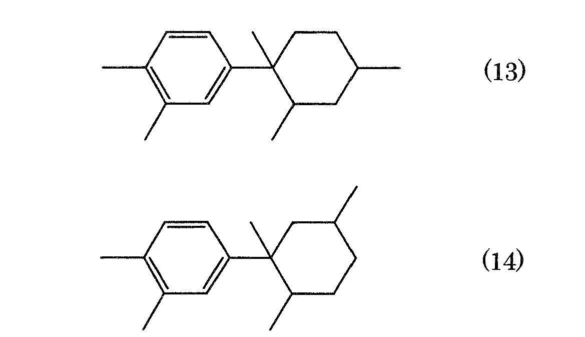 These by-products are isomers in terms of position of the double bond of trimethylcyclohexene serving as a starting material. The by-product formation is considerably predominant when a liquid-form catalyst such as sulfuric acid or hydrogen fluoride is used. When H-type zeolite or NH₄-type zeolite, particularly MFI-type zeolite (e.g., H-type mordenite, NH₄-type mordenite, H-type β-zeolite, or ZSM-5) or MTW-type zeolite (e.g., ZSM-12), is used, by-production of non-target isomers may be prevented. Since these zeolites have a pore size of 0.5 to 0.8 nm, formation of considerably bulky isomers is prevented by virtue of such a small pore size. Therefore, the target 1-(3,4-dimethylphenyl)-1,3,4-trimethylcyclohexane can be predominantly produced, regardless of the position of the double bond in trimethylcyclohexene serving as a starting material.

The acid catalyst is preferably used in an amount (ratio by weight) of 0.0001 to 10,000 with respect to the amount of alkylcyclohexene, more preferably 0.001 to 1,000. An excessively small amount results in poor catalytic effect, and an excessive amount is not economically preferred. When alkylcyclohexanol is used instead of alkylcyclohexene, the amount of catalyst often increases as compared with the case of alkylcyclohexene. In this case, the amount of catalyst (ratio by weight) is preferably 0.0001 to 10,000 with respect to the amount of alkylcyclohexanol, more preferably 0.001 to 1,000. For example, in condensation between toluene and 1,4-dimethylcyclohexene, an acid catalyst (protonic acid or Lewis acid) is preferably used in an amount of 0.1 mol to 100 mol, with respect to 1 mol of 1,4-dimethylcyclohexene. A solid acidic catalyst such as a cation-exchange resin or zeolite is preferably used in an amount of 0.1 to 10 g, with respect to 1 g of 1,4-dimethylcyclohexene. When the catalytic activity of zeolite has been lowered, the catalyst may be reactivated through a conventional method, for example, calcination in air.

The condensation is performed in a variety of reaction modes both in a batch manner and a continuous flow manner such as an immobilized bed, a suspension bed, and a uniform bath. Among them, an immobilized bed is preferred, since the catalyst is readily separated and recovered. When a solid acid catalyst is used in a flow manner, alkylcyclohexene or alkylcyclohexanol is preferably fed continuously to a flow reactor, such that the weight hourly space velocity (WHSV) is regulated to 0.001 to 10 hr⁻¹. The reaction temperature, which depends on the type and amount of catalyst and the type and concentration of reaction substrate, is -50 to 250°C. In the case of sulfuric acid or hydrochloric acid, a reaction temperature of 0 to 50°C is preferred. In the case of hydrogen fluoride, a reaction temperature of -50 to 50°C is preferred. In the case of zeolite or an ion-exchange resin, a reaction temperature of 50 to 200°C is preferred. When the reaction temperature is higher than the upper limit of each case, side reaction to form isomers and other products is promoted, whereas when the reaction temperature is lower than the lower limit of each case, sufficient reaction rate fails to be attained. The condensation may be performed under any of normal pressure, reduced pressure, and elevated pressure. When the starting materials or the solvent employed in the reaction have a boiling point higher than the reaction temperature, the reaction is preferably performed under a pressurized condition of 0.1 to 10 MPa. Pressurizing is preferably performed through introducing an inert gas such as nitrogen or argon to the reaction system. The reaction time is preferably 10 minutes to 20 hours, more preferably 10 minutes to 3 hours, when a batch manner is employed.

In some cases, the (alkylphenyl)alkylcyclohexane formed in the aforementioned condensation reaction is a mixture of cis- and trans-isomers. A target product may be separated from such an isomer mixture through a conventional technique such as distillation, crystallization, or a column process, whereby starting materials and by-products are removed. The isomer mixture of (alkylphenyl)alkylcyclohexane may be separated into respective isomers, or the mixture itself may be used as a starting material in a subsequent step (simultaneous dehydrogenation/dealkylation).

Through dehydrogenation of the cyclohexane ring of the thus-produced (alkylphenyl)alkylcyclohexane in the presence of a catalyst and simultaneously through dealkylation of R³, an alkylbiphenyl represented by formula (7): 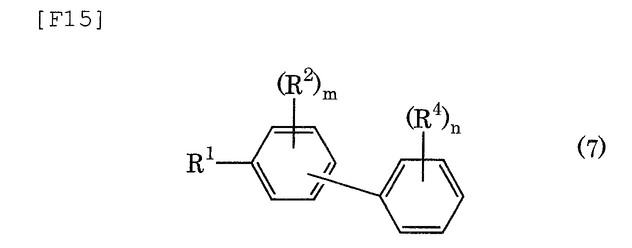 (wherein R¹, R², R⁴, m, and n have the same meanings as defined above, wherein when no R² is present or R² is present at the o-position with respect to R¹, the benzene ring having R⁴ is present at the p-position with respect to R¹, and when R² is present at the m-position with respect to R¹, the benzene ring having R⁴ is present at the m-position with respect to R¹) can be produced.

Examples of the simultaneous dehydrogenation/dealkylation catalyst include metals such as platinum, palladium, rhodium, rhenium, ruthenium, iron, chromium, cobalt, nickel, molybdenum, copper, zinc, and iridium; compounds containing the metals; zeolite; alumina; silica-alumina; and mixtures thereof. Of these, compounds containing chromium or zinc are particularly preferred from the viewpoints of catalytic activity and selectivity. Specific examples of the compound containing chromium or zinc include chromium(III) oxide, zinc oxide, Cu-Cr, Cu-Zn, and Zn-Cr. To these compounds, an alkali metal such as lithium, sodium, or potassium; or an alkaline earth metal such as magnesium or calcium may be added. Among them, potassium is particularly preferred. In use, these catalysts may be supported on a carrier such as carbon, alumina, zirconia, or silica-alumina. The catalyst may be subjected to a preliminary treatment which is conventionally performed, for example, heating under a stream of oxidizing gas (e.g., air) or reducing gas (e.g., hydrogen). When the catalytic activity has been lowered, the catalyst can be reactivated through the same treatment. No particular limitation is imposed on the method for preparing the catalyst. For example, a catalyst may be produced through the impregnation method including dissolving a metal salt (e.g., nitrate or acetate) in water, impregnating a carrier such as γ-alumina with the solution, drying, and calcinating in air at 300°C to 600°C. When the flow manner is employed, the weight hourly space velocity (WHSV) is preferably 0.0001 to 100 hr⁻¹, more preferably 0.001 to 10 hr⁻¹.

The mode of simultaneous dehydrogenation/dealkylation is preferably an immobilized bed-flow manner. The reaction temperature is preferably 200 to 600°C, more preferably 350 to 550°C. When the temperature is lower than the lower limit, reaction does not sufficiently proceed, whereas when the temperature is higher than the upper limit, selectivity decreases. The simultaneous dehydrogenation/dealkylation may be performed under any of normal pressure, reduced pressure, and elevated pressure. During reaction, nitrogen, hydrogen, or steam may also be passed. A flow of hydrogen is preferred, since deterioration of the catalyst can be prevented. The (alkylphenyl)alkylcyclohexane as is may be fed to a reactor. Alternatively, a solvent inert to the reaction such as benzene or toluene may also be used. Yet alternatively, the alkylcyclohexane which has been by-produced in the aforementioned production of alkylcyclohexene (formula (4)) through partial catalytic hydrogenation of alkylbenzene (formula (9)) and which is represented by formula (15): 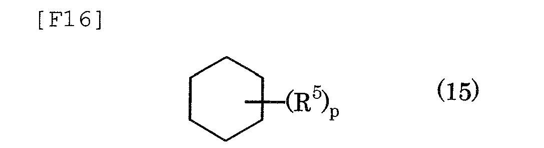 (wherein R⁵ and p have the same meanings as defined above) may be employed as a solvent.

The by-product of the aforementioned alkylcyclohexene production step is substantially sole alkylcyclohexane. The by-produced alkylcyclohexane is converted to an alkylbenzene (formula (9)) in the simultaneous dehydrogenation/dealkylation step, and reused as a starting material for producing alkylcyclohexene. Therefore, when the alkylcyclohexene production step, the alkylcyclohexene condensation step, and the (alkylphenyl)alkylcyclohexane simultaneous dehydrogenation/dealkylation step are sequentially performed, loss of the starting alkylbenzene (formula (9)) used in the alkylcyclohexene production step is avoided, whereby alkylbiphenyl can be produced through a economical process.

The thus-produced alkylbiphenyl may be separated through a conventional technique such as distillation, cryslallization, or a column process. From the alkylbiphenyl, the corresponding biphenyl dicarboxylic acid, biphenyltetracarboxylic acid, and biphenyltetracarboxylic anhydride can be produced through a known method.

### Examples

The present invention will next be described in detail by way of examples, which should not be construed as limiting the invention thereto. Conversion, the selectivity, and yield were determined through gas chromatography according to the internal standard method.

### Example 1

To a 3,000-mL three-neck glass flask equipped with a thermometer and a condenser, toluene (2,700 g), concentrated sulfuric acid (15 g), and 1,4-dimethylcyclohexene (55 g) were fed, and the reaction mixture was maintained at -20°C. The mixture was allowed to react for 5 hours, and the organic layer was separated. The separated organic layer was sequentially washed twice with distilled water (500 mL), once with 3% aqueous sodium hydrogencarbonate solution, and twice with distilled water. Through gas chromatographic analysis of the organic layer, the conversion of 1,4-dimethylcyclohexene was found to be 96%, and the selectivity to 1-(4-methylphenyl)-1,4-dimethylcyclohexane was found to be 92%.

### Example 2

Hydrogen fluoride (130 g) and toluene (100 g) were placed in a 500-mL autoclave made of Hastelloy C, and the mixture was cooled to -27°C. To the mixture, a liquid mixture of 1,4-dimethylcyclohexene (10 g) and toluene (100 g) was added over 1 hour. After completion of addition, the mixture was allowed to stand for 20 minutes, and liquid was separated to recover hydrogen fluoride. During reaction, the pressure in the system was 0.2 MPa or less. The separated organic layer was sequentially washed thrice with distilled water (200 mL), once with 5% aqueous sodium hydrogencarbonate solution, and twice with distilled water. Through gas chromatographic analysis of the organic layer, the conversion of 1,4-dimethylcyclohexene was found to be 100%, and the selectivity to 1-(4-methylphenyl)-1,4-dimethylcyclohexane was found to be 28.5%.

### Example 3

Toluene (250 g), H-type mordenite (product of Tosoh Corporation, HSZ-640HOD1C) (5 g), and 1,4-dimethylcyclohexene (25 g) were added to a 500-mL electromagnetic-stirring-type autoclave made of SUS304 equipped with a condenser and a thermometer. The system in the autoclave was purged with nitrogen gas. After the system had been filled with nitrogen gas (3 MPa), the mixture was heated at 180°C with stirring. During reaction, the pressure in the system was elevated to 4 MPa. After reaction for 3 hours, the reaction mixture was recovered, and the catalyst was removed through filtration. Through gas chromatographic analysis of the organic layer, the conversion of 1,4-dimethylcyclohexene was found to be 85.8%, and the selectivity to 1-(4-methylphenyl)-1,4-dimethylcyclohexane was found to be 59.5%.

### Example 4

The reaction procedure of Example 3 was repeated, except that the reaction was performed at 110°C and under normal pressure for 50 hours under toluene reflux conditions. After completion of reaction, the reaction mixture was recovered, and the catalyst was removed through filtration. Through gas chromatographic analysis of the organic layer, the conversion of 1,4-dimethylcyclohexene was found to be 70.1%, and the selectivity to 1-(4-methylphenyl)-1,4-dimethylcyclohexane was found to be 63.2%.

### Example 5

Toluene (5.0 g), H-type β-zeolite (product of Tosoh Corporation, HSZ-930HOD1A) (0.1 g), and 1,4-dimethylcyclohexene (0.25 g) were added to a 50-mL autoclave made of SUS304. The system in the autoclave was purged with nitrogen gas. After the system had been filled with nitrogen gas (2 MPa), the mixture was heated at 200°C with stirring. During reaction, the pressure in the system was elevated to 3 MPa. After reaction for 20 hours, the reaction mixture was recovered, and the catalyst was removed through filtration. Through gas chromatographic analysis of the organic layer, the conversion of 1,4-dimethylcyclohexene was found to be 77.5%, and the selectivity to 1-(4-methylphenyl)-1,4-dimethylcyclohexane was found to be 62.5%.

### Example 6

The reaction procedure of Example 5 was repeated, except that H-type SAPO-5 zeolite (prepared with reference to a method disclosed in the description (H. Robson, "Verified Syntheses of Zeoritic Materials," Elsevier Science B.V. p. 93)) (0.1 g) was used. During reaction, the pressure in the system was elevated to 3 MPa. After completion of reaction, the reaction mixture was recovered, and the catalyst was removed through filtration. Through gas chromatographic analysis of the organic layer, the conversion of 1,4-dimethylcyclohexene was found to be 42.6%, and the selectivity to 1-(4-methylphenyl)-1,4-dimethylcyclohexane was found to be 60.6%.

### Example 7

To a 3,000-mL three-neck glass flask equipped with a thermometer and a condenser, o-xylene (2,000 g), concentrated sulfuric acid (24 g), and 1,2,4-trimethylcyclohexene (50 g) were fed, and the reaction mixture was maintained at 20°C. The mixture was allowed to react for 1 hour, and the organic layer was separated. The separated organic layer was sequentially washed twice with distilled water (50 mL), once with 3% aqueous sodium hydrogencarbonate solution, and twice with distilled water. Through gas chromatographic analysis of the organic layer, the conversion of 1,2,4-trimethylcyclohexene was found to be 87%, and the selectivity to 1-(3,4-dimethylphenyl)-1,3,4-trimethylcyclohexane was found to be 43%.
After removal of low-boiling-point fractions from the reaction mixture, the residue was distilled by means of a distillation tower (number of theoretical plates: 10), to thereby obtain two fractions: percent recovered (4 torr) 140°C and 142°C. Through analyses (GC-MS, ¹H-NMR, and ¹³C-NMR), the two fractions were identified to the following isomers (isomers 1 and 2) of 1-(3,4-dimethylphenyl)-1,3,4-trimethylcyclohexane.

### Isomer 1

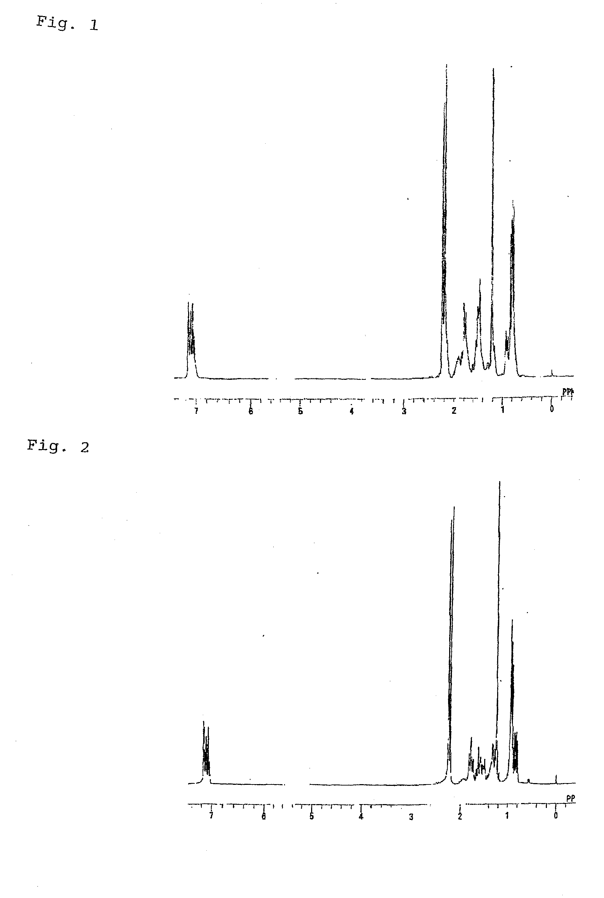

GC-MS: M+=230
¹H-NMR (solvent: CDCl₃) , δ (ppm) :
7.0 to 7.2 (m, 3H, aromatic ring H)
2.2 and 2.3 (s, 3H, aromatic ring-bound methyl H)
1.2 (s, 3H, cyclohexane ring quaternary carbon-bound methyl H)
0.8 to 0.9 (m, 6H, cyclohexane ring-bound methyl H)
1.7 to 2.0 (m, 2H, cyclohexane ring methine H)
1.3 to 1.6 (m, 6H, cyclohexane ring methylene H)
¹³C-NMR (solvent: CDCl₃), δ (ppm) :
133, 135, 151 (aromatic ring quaternary C)
122, 126, 129 (aromatic ring methine C)
41, 31, 29 (cyclohexane ring methylene C)
37 (cyclohexane ring quaternary C)
32, 31 (cyclohexane ring methine C)
19, 20 (aromatic ring-bound methyl C)
11, 20 (cyclohexane ring methine-bound methyl C)
26 (cyclohexane ring quaternary carbon-bound methyl C) Fig. 1 shows a ¹H-NMR spectrum of isomer 1.

### Isomer 2

GC-MS: M+=230
¹H-NMR (solvent: CDCl₃) , δ (ppm) :
7.0 to 7.2 (m, 3H, aromatic ring H)
2.2 and 2.3 (s, 3H, aromatic ring-bound methyl H)
1.2 (s, 3H, cyclohexane ring quaternary carbon-bound methyl H)
0.9 to 1.0 (m, 6H, cyclohexane ring-bound methyl H)
0.9 to 1.5 (m, 2H, cyclohexane ring methine H)
1.2 to 1.9 (m, 6H, cyclohexane ring methylene H)
¹³C-NMR (solvent: CDCl₃) , δ (ppm) :
133, 136, 150 (aromatic ring quaternary C)
122, 126, 129 (aromatic ring methine C)
47, 38, 32 (cyclohexane ring methylene C)
37 (cyclohexane ring quaternary C)
39, 35 (cyclohexane ring methine C)
19, 20 (aromatic ring-bound methyl C)
11, 20 (cyclohexane ring methine-bound methyl C)
25 (cyclohexane ring quaternary carbon-bound methyl C) Fig. 2 shows a ¹H-NMR spectrum of isomer 2.

### Example 8

To a 200-mL three-neck glass flask equipped with a thermometer and a condenser, o-xylene (100 g), H-type mordenite (silica/alumina ratio: 200, product of Tosoh, HSZ-690HOA) (25 g), and 1,2,4-trimethylcyclohexene (5 g) were fed, and the reaction mixture was heated under normal pressure to a flux temperature (150°C). After reaction for 1 hour, the catalyst was separated from the mixture. Through gas chromatographic analysis of the reaction mixture, the conversion of 1,2,4-trimethylcyclohexene was found to be 95%, and the selectivity to 1-(3,4-dimethylphenyl)-1,3,4-trimethylcyclohexane was found to be 72%.

### Example 9 to 12

The reaction procedure of Example 8 was repeated, except that zeolites listed in Table 1 were used instead of H-type mordenite. The results are shown in Table 1.
[Table 1]

**Table 1**

| Examples | Zeolite | Conversion (%) of 1,2,4- trimethyl- cyclohexene | Selectivity (%) to 1-(3,4-dimethylphenyl)-1,3,4-trimethylcyclohexane |
|---|---|---|---|
| 9 | ZSM-5 NH₄-type | 79 | 41 |
| 10 | ZSM-5 H-type | 57 | 30 |
| 11 | ZSM-12 H-type | 85 | 26 |
| 12 | H-β | 46 | 3 |

### Example 13

To a 300-mL three-neck glass flask equipped with a thermometer and a condenser, o-xylene (10 g), concentrated sulfuric acid (0.3 g), and 1,4-dimethylcyclohexene (0.5 g) were fed, and the reaction mixture was maintained at 20°C. The mixture was allowed to react for 1 hour, and the organic layer was separated. The separated organic layer was sequentially washed twice with distilled water (30 mL), once with 3% aqueous sodium hydrogencarbonate solution, and twice with distilled water. Through gas chromatographic analysis of the organic layer, the conversion of 1,4-dimethylcyclohexene was found to be 94%, and the selectivity to 1-(3,4-dimethylphenyl)-1,4-dimethylcyclohexane was found to be 71%.

### Example 14

The reaction procedure of Example 13 was repeated, except that m-xylene was used instead of o-xylene. Through gas chromatographic analysis of the organic layer, the conversion of 1,4-dimethylcyclohexene was found to be 92%, and the selectivity to 1-(3,5-dimethylphenyl)-1,4-dimethylcyclohexane was found to be 11%.

### Example 15

The reaction procedure of Example 13 was repeated, except that ethylbenzene was used instead of o-xylene. Through gas chromatographic analysis of the organic layer, the conversion of 1,4-dimethylcyclohexene was found to be 89%, and the selectivity to 1-(4-ethylphenyl)-1,4-dimethylcyclohexane was found to be 60%.

### Example 16

The reaction procedure of Example 13 was repeated, except that propylbenzene was used instead of o-xylene. Through gas chromatographic analysis of the organic layer, the conversion of 1,4-dimethylcyclohexene was found to be 93%, and the selectivity to 1-(4-propylphenyl)-1,4-dimethylcyclohexane was found to be 31%.

### Example 17

The reaction procedure of Example 13 was repeated, except that isopropylbenzene was used instead of o-xylene. Through gas chromatographic analysis of the organic layer, the conversion of 1,4-dimethylcyclohexene was found to be 85%, and the selectivity to 1-(4-isopropylphenyl)-1,4-dimethylcyclohexane was found to be 28%.

### Example 18

The reaction procedure of Example 13 was repeated, except that 1,2,3-trimethylbenzene was used instead of o-xylene. Through gas chromatographic analysis of the organic layer, the conversion of 1,4-dimethylcyclohexene was found to be 97%, and the selectivity to 1-(3,4,5-trimethylphenyl)-1,4-dimethylcyclohexane was found to be 46%.

### Example 19

To a 100-mL three-neck glass flask equipped with a thermometer and a condenser, m-xylene (10 g), concentrated sulfuric acid (0.3 g), and 1,2,4-trimethylcyclohexene (0.5 g) were fed, and the reaction mixture was maintained at 20°C. The mixture was allowed to react for 1 hour, and the organic layer was separated. The separated organic layer was sequentially washed twice with distilled water (30 mL), once with 3% aqueous sodium hydrogencarbonate solution, and twice with distilled water. Through gas chromatographic analysis of the organic layer, the conversion of 1,2,4-trimethylcyclohexene was found to be 98%, and the selectivity to 1-(3,5-dimethylphenyl)-1,3,4-trimethylcyclohexane was found to be 17%.

### Example 20

The reaction procedure of Example 19 was repeated, except that 1,2,3-trimethylbenzene was used instead of m-xylene. Through gas chromatographic analysis of the organic layer, the conversion of 1,2,4-trimethylcyclohexene was found to be 95%, and the selectivity to 1,3,4-trimethyl-1-(3,4,5-trimethylphenyl)cyclohexane was found to be 53%.

### Example 21

To a 300-mL three-neck glass flask equipped with a thermometer and a condenser, toluene (65 g), concentrated sulfuric acid (3.5 g), and 2,5-dimethylcyclohexanol (2.5 g) were fed, and the reaction mixture was maintained at 20°C. The mixture was allowed to react for 1 hour, and the organic layer was separated. The separated organic layer was sequentially washed twice with distilled water (20 mL), once with 3% aqueous sodium hydrogencarbonate solution, and twice with distilled water. Through gas chromatographic analysis of the organic layer, the conversion of 2,5-dimethylcyclohexanol was found to be 88%, and the selectivity to 1-(4-methylphenyl)-1,4-dimethylcyclohexane was found to be 74%.

### Example 22

To a 100-mL three-neck glass flask equipped with a thermometer and a condenser, o-xylene (20 g), concentrated sulfuric acid (3 g), and 3,4-dimethylcyclohexanol (2 g) were fed, and the reaction mixture was maintained at 20°C. The mixture was allowed to react for 1 hour, and the organic layer was separated. The separated organic layer was sequentially washed twice with distilled water (20 mL), once with 3% aqueous sodium hydrogencarbonate solution, and twice with distilled water. Through gas chromatographic analysis of the organic layer, the conversion of 3,4-dimethylcyclohexanol was found to be 68%, and the selectivity to 1-(3,4-dimethylphenyl)-1,2-dimethylcyclohexane was found to be 74%.

### Example 23

A glass reactor tube (250 mm (length) × 12 mmφ (inner diameter) having a sheath tube for temperature measurement was employed. A material feed line and a hydrogen gas feed line were attached to the upper section of the reactor tube, and a Dimroth condenser, a flask for collecting a reaction mixture, and a vent line for discharging gas were attached to the lower section of the reactor tube. A catalyst, 10% Cr₂O₃-1% K/Al₂O₃, (8 g) was placed in the reactor tube, and the reactor tube was heated at 450°C, while hydrogen was caused to flow in the tube at 50 mL/min. Through the material feed line, a isomer mixture of 1-(3,4-dimethylphenyl)-1,3,4-trimethylcyclohexane (isomer 1/isomer 2 = 60/40 (ratio by weight)) produced in Example 7 in the form of 10 wt.% benzene solution was fed to the reactor tube at 10 g/hr for starting reaction. At hour 2 to hour 5 after the start of the reaction, the reaction mixture was sampled and analyzed through GC and NMR. As a result, 3,4,3',4'-tetramethylbiphenyl was found to be produced at a yield of 92%, and no other tetramethylbiphenyl isomers were observed.

### Example 24

### Step 1

Water (100 g), a 5%Ru alumina catalyst (1 g), zinc sulfate (0.06 g), and p-xylene (200 g) were fed to a 500-mL electromagnetic-stirring-type autoclave made of SUS316 equipped with a thermometer. The system was pressurized with hydrogen to 5 MPa, and the mixture was allowed to react at 150°C for 4 hours. After cooling, the oil layer was separated from the reaction mixture and analyzed through gas chromatography. The conversion of p-xylene was found to be 80%, and the selectivity to 1,4-dimethylcyclohexene and to 1,4-dimethylcyclohexane were found to be 39% and 61%, respectively. Through distillation, 1,4-dimethylcyclohexene and 1,4-dimethylcyclohexane were separated from the produced reaction mixture.

### Step 2

To a 3,000-mL three-neck glass flask equipped with a thermometer and a condenser, toluene (2,700 g), concentrated sulfuric acid (15 g), and 1,4-dimethylcyclohexene (55 g) were fed, and the reaction mixture was maintained at -20°C. The mixture was allowed to react for 5 hours, and the organic layer was separated. The separated organic layer was sequentially washed twice with distilled water (500 mL), once with 3% aqueous sodium hydrogencarbonate solution, and twice with distilled water. Through gas chromatographic analysis of the organic layer, the conversion of 1,4-dimethylcyclohexene was found to be 96%, and the selectivity to 1-(4-methylphenyl)-1,4-dimethylcyclohexane was found to be 92%. Through distillation, 1-(4-methylphenyl)-1,4-dimethylcyclohexane was separated from the produced reaction mixture.

### Step 3

A glass reactor tube (250 mm (length) × 12 mmφ (inner diameter) having a sheath tube for temperature measurement was employed. A material feed line and a hydrogen gas feed line were attached to the upper section of the reactor tube, and a Dimroth condenser, a flask for collecting a reaction mixture, and a vent line for discharging gas were attached to the lower section of the reactor tube. A catalyst, 10% Cr₂O₃-1% K/Al₂O₃, (8 g) was placed in the reactor tube, and the reactor tube was heated at 450°C, while hydrogen was caused to flow in the tube at 50 mL/min. Through the material feed line, the aforementioned 1-(4-methylphenyl)-1,4-dimethylcyclohexane in the form of 10% benzene solution was fed to the reactor tube at 10 g/hr for starting reaction. At hour 2 to hour 5 after the start of the reaction, the reaction mixture was sampled and analyzed. As a result, the conversion of 1-(4-methylphenyl)-1,4-dimethylcyclohexane was found to be 100%, and the yield of 4,4'-dimethylbiphenyl was found to be 78%.

### Example 25

The reaction procedure of Example 24 was repeated, except that the catalyst employed in Step 3 was changed to zinc oxide. The conversion of 1-(4-methylphenyl)-1,4-dimethylcyclohexane was found to be 70%, and the yield of 4,4'-dimethylbiphenyl was found to be 49%.

### Example 26

The reaction procedure of Example 24 was repeated, except that benzene employed as a solvent employed in Step 3 was changed to 1,4-dimethylcyclohexane which had been by-produced in Step 1. The conversion of 1-(4-methylphenyl)-1,4-dimethylcyclohexane was found to be 80%, and the yield of 4,4'-dimethylbiphenyl was found to be 45%. The conversion of 1,4-dimethylcyclohexane was found to be 14%, and the yield of p-xylene was found to be 14%.

### Example 27

### Step 1

Water (100 g), a 5%Ru alumina catalyst (2 g), and zinc sulfate (0.06 g), and pseudocumene (200 g) were fed to a reactor as employed in Example 24. The system was pressurized with hydrogen to 10 MPa, and the mixture was allowed to react at 160°C for 2.5 hours. After cooling, the oil layer was separated from the reaction mixture and analyzed through gas chromatography. The conversion of pseudocumene was found to be 65%, and the selectivity to 1,2,4-trimethylcyclohexene and to 1,2,4-trimethylcyclohexane were found to be 31% and 69%, respectively. Through distillation, a 1,2,4-trimethylcyclohexene isomer mixture was separated from the produced reaction mixture.

### Step 2

o-Xylene (100 g), H-type mordenite (silica/alumina ratio: 200, product of Tosoh, HSZ-690HOA) (25 g), and a 1,2,4-trimethylcyclohexene isomer mixture (5 g) were fed to a reactor as employed in Example 24. The reaction mixture was heated under normal pressure to a flux temperature (150°C). After reaction for 1 hour, the catalyst was separated from the mixture. Through gas chromatographic analysis of the reaction mixture, the conversion of 1,2,4-trimethylcyclohexene was found to be 95%, and the selectivity to 1-(3,4-dimethylphenyl)-1,3,4-trimethylcyclohexane was found to be 72%. Through distillation, 1-(3,4-dimethylphenyl)-1,3,4-trimethylcyclohexane was separated from the produced reaction mixture.

### Step 3

The reaction procedure of Example 24 was repeated, except that 1-(3,4-dimethylphenyl)-1,3,4-trimethylcyclohexane was employed as a starting material. The conversion of 1-(3,4-dimethylphenyl)-1,3,4-trimethylcyclohexane was found to be 94%, and the yield of 3,4,3',4'-tetramethylbiphenyl was found to be 85%.

### Example 28

Water (10 g), a 5%Ru alumina catalyst (0.2 g), and mesitylene (20 g) were fed to a 100-mL electromagnetic-stirring-type autoclave made of SUS316 equipped with a thermometer. The system was pressurized with hydrogen to 10 MPa, and the mixture was allowed to react at 150°C for 2 hours. After cooling, the oil layer was separated from the reaction mixture and analyzed through gas chromatography. The conversion of mesitylene was found to be 49%, and the selectivity to 1,3,5-trimethylcyclohexene and to 1,3,5-trimethylcyclohexane were found to be 21% and 79%, respectively.
To a 100-mL three-neck glass flask equipped with a thermometer and a condenser, o-xylnene (10 g), sulfuric acid (0.3 g), and 1,3,5-trimethylcyclohexene (0.5 g) were fed, and the reaction mixture was maintained at 20°C. The mixture was allowed to react for 1 hour, and the organic layer was separated. The separated organic layer was sequentially washed twice with distilled water (30 mL), once with 3% aqueous sodium hydrogencarbonate solution, and twice with distilled water. Through gas chromatographic analysis of the organic layer, the conversion of 1,3,5-trimethylcyclohexene was found to be 92%, and the selectivity to 1-(3,4-dimethylphenyl)-1,3,5-trimethylcyclohexane was found to be 15%.

### Industrial Applicability

According to the method of the present invention, an (alkylphenyl)alkylcyclohexane and an alkylbiphenyl of interest can be efficiently produced from an inexpensive material at high selectivity, without performing a post treatment. Thus, the method of the invention is remarkably advantageous in the industry.

## Claims

1. A method for producing an (alkylphenyl)alkylcyclohexane represented by formula (6): 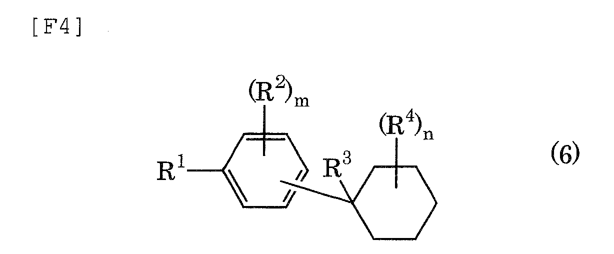 (wherein R¹ represents a C1-C4 alkyl group; R² represents a C1-C4 alkyl group which is identical to or different from R¹; R³ represents a C1-C4 alkyl group; R⁴ represents a C1-C4 alkyl group which is identical to or different from R³; m is an integer of 0 to 2; and n is an integer of 1 to 5, wherein when no R² is present or R² is present at the o-position with respect to R¹, the cyclohexane ring is present at the p-position with respect to R¹, and when R² is present at the m-position with respect to R¹, the cyclohexane ring is present at the m-position with respect to R¹), the method comprising a step of condensing an alkylbenzene represented by formula (3): 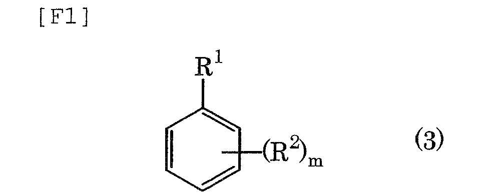 (wherein R¹, R², and m have the same meanings as defined above, wherein when m is 1, R² is present at the o- or m-position with respect to R¹, and when m is 2, two R²_{S} are present at positions which are different from each other and are ortho with respect to R¹) with an alkylcyclohexene represented by formula (4): 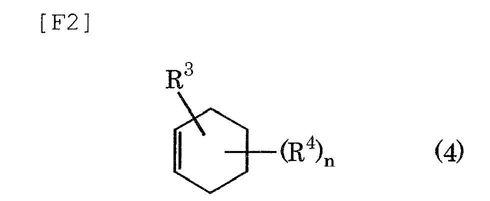 (wherein R³, R⁴, and n have the same meanings as defined above, wherein when n is an integer of 2 to 5, a plurality of R⁴s may be identical to or different from one another, except that R³ and R⁴ or two R⁴s are bonded to one carbon atom) or with an alkylcyclohexanol represented by formula (5): 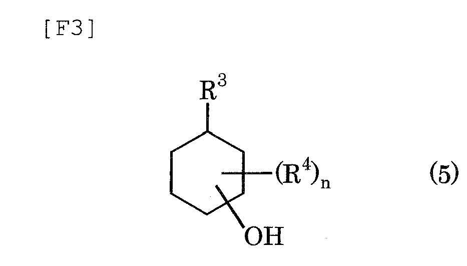 (wherein R³, R⁴, and n have the same meanings as defined above) in the presence of an acid catalyst.

2. A production method as described in claim 1, wherein the acid catalyst is at least one species selected from the group consisting of sulfuric acid, hydrochloric acid, phosphoric acid, polyphosphoric acid, hydrogen fluoride, hydroborofluoric acid, boron trifluoride, aluminum trichloride, aluminum tribromide, gallium trichloride, gallium tribromide, iron trichloride, antimony pentachloride, tin tetrachloride, titanium tetrachloride, and zinc chloride.

3. A production method as described in claim 1, wherein the acid catalyst is at least one species selected from the group consisting of a cation-exchange resin, silica-alumina, and zeolite.

4. A production method as described in claim 1, wherein the alkylcyclohexene represented by formula (4) is produced through catalytic partial hydrogenation of an alkylbenzene represented by formula (9): 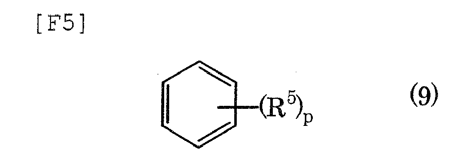 (wherein R⁵ represents a C1-C4 alkyl group, and p is an integer of 2 to 6, wherein a plurality of R⁵s may be identical to or different from one another).

5. A production method as described in claim 1, wherein, in formulas (3) to (6), each of R¹, R², R³, and R⁴ is a methyl group.

6. A production method as described in claim 1, wherein 1-(4-methylphenyl)-1,4-dimethylcyclohexane is produced from toluene as the alkylbenzene represented by formula (3) and 1,4-dimethylcyclohexene as the alkylcyclohexene represented by formula (4).

7. A production method as described in claim 6, wherein the acid catalyst is at least one species selected from the group consisting of sulfuric acid, hydrogen fluoride, H-type mordenite, and H-type β-zeolite.

8. A production method as described in claim 1, wherein 1-(3,4-dimethylphenyl)-1,3,4-trimethylcyclohexane is produced from o-xylene as the alkylbenzene represented by formula (3) and 1,2,4-trimethylcyclohexene as the alkylcyclohexene represented by formula (4).

9. A production method as described in claim 8, wherein the acid catalyst is at least one species selected from the group consisting of sulfuric acid, H-type mordenite, NH₄-type mordenite, H-type or NH₄-type MFI zeolite, H-type or NH₄-type MTW zeolite, and H-type β-zeolite.

10. A method for producing an alkylbiphenyl represented by formula (7): 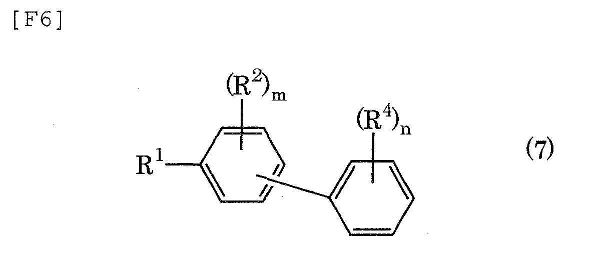 (wherein R¹, R², R⁴, m, and n have the same meanings as defined above, wherein when no R² is present or R² is present at the o-position with respect to R¹, the benzene ring having R⁴ is present at the p-position with respect to R¹, and when R² is present at the m-position with respect to R¹, the benzene ring having R⁴ is present at the m-position with respect to R¹), the method comprising a step of simultaneously dehydrogenating and dealkylating, in the presence of a catalyst, an (alkylphenyl)alkylcyclohexane which is produced through a production method as recited in claim 1 and which is represented by formula (6).

11. A production method as described in claim 10, wherein the catalyst is at least one species selected from the group consisting of a metal such as platinum, palladium, rhodium, rhenium, ruthenium, iron, chromium, cobalt, nickel, molybdenum, copper, zinc, or iridium; a compound containing the metal, zeolite, alumina, and silica-alumina.

12. A production method as described in claim 10, wherein the alkylcyclohexane which has been by-produced in the partial catalytic hydrogenation of alkylbenzene as recited in claim 4 and which is represented by formula (15): 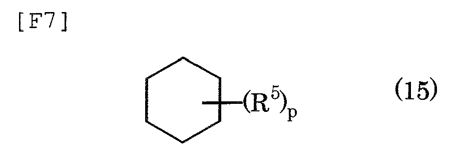 (wherein R⁵ and p have the same meanings as defined above) is employed as a solvent.

13. A production method as described in claim 10, wherein the alkylcyclohexane represented by formula (15) is dehydrogenated in the step of simultaneous dehydrogenation and dealkylation, to thereby transform into an alkylbenzene represented by formula (9): 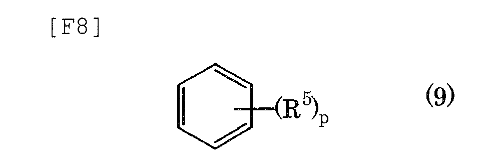 (wherein R⁵ and p have the same meanings as defined above).

14. A production method as described in claim 10, wherein the alkylbenzene which has been produced according to claim 13 and which is represented by formula (9) is employed as a starting material in a step of producing an alkylcyclohexene as recited in claim 4.

15. A production method as described in claim 10, which further comprises a step of oxidizing the alkylbiphenyl, to thereby form a corresponding biphenyl polycarboxylic acid.

16. A production method as described in claim 15, wherein the biphenyl polycarboxylic acid is 4,4'-biphenyldicarboxylic acid or 3,4,3',4'-biphenyltetracarboxylic acid.

17. A production method as described in claim 16, which further comprises a step of dehydrating the 3,4,3',4'-biphenyltetracarboxylic acid to form 3,4,3',4'-biphenyltetracarboxylic anhydride.

18. An (alkylphenyl)alkylcyclohexane represented by formula (8) : 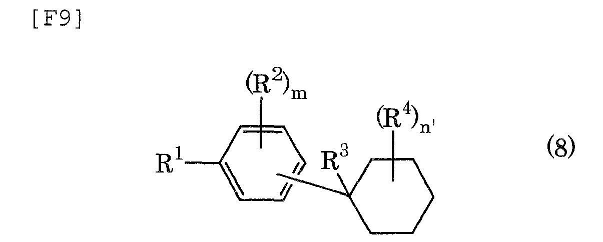 (wherein R¹ represents a C1-C4 alkyl group; R² represents a C1-C4 alkyl group which is identical to or different from R¹; m is an integer of 0 to 2, wherein when m is 1, R² is present at the o- or m-position with respect to R¹, and when m is 2, two R²s are present at positions which are different from each other and are ortho with respect to R¹; R³ represents a C1-C4 alkyl group; R⁴ represents a C1-C4 alkyl group which is identical to or different from R³; wherein when no R² is present or R² is present at the o-position with respect to R¹, the cyclohexane ring is present at the p-position with respect to R¹, and when R² is present at the m-position with respect to R¹, the cyclohexane ring is present at the m-position with respect to R¹; n' is an integer of 2 to 5; wherein a plurality of R⁴s may be identical to or different from one another, except that two R⁴s are bonded to one carbon atom).

19. An (alkylphenyl)alkylcyclohexane as described in claim 18, which is 1-(3,4-dimethylphenyl)-1,3,4-trimethylcyclohexane, represented by formula (2).
